# EUROPÄISCHE PATENTANMELDUNG

(11) **EP 1 972 292 A1**
(43) Veröffentlichungstag der Anmeldung: **24.09.2008**
(21) Anmeldenummer: 07005977.9
(22) Anmeldetag: 22.03.2007
(51) Int. Cl.: A61B 19/00, A61L 2/00

(54) **Verfahren zum maschinellen Aufbereiten eines wiederverwendbaren Medizinproduktes**

(71) Anmelder: Chemische Fabrik Dr. Weigert GmbH & Co. KG, 20539 Hamburg (DE); Antonio Matachana, S.A., 08018 Barcelona (ES)
(72) Erfinder: Kamer, Markus, 21465 Reinbek (DE); Wagemann, Wolfgang, 22967 Tremsbüttel (DE); von dem Hagen, Tronje, 23554 Lübeck (DE); Aramburu, Manuel Matachana, 08328 Alella (ES)
(74) Vertreter: Glawe, Delfs, Moll

(57) **Zusammenfassung**

Gegenstand der Erfindung ist ein Verfahren zum maschinellen Aufbereiten eines widerverwendbaren Medizinprodukts. Das Verfahren weist folgende Schritte auf:
a) Einlegen des Medizinprodukts (11) in eine verschließbare Kassette (14), die Anschlüsse für die Zu- und Abfuhr von Aufbereitungsmedium aufweist,
b) Verschließen der Kassette (14),
c) Zuführen von frischem Aufbereitungsmedium in den oder die Zuführanschlüsse der Kassette (14), wobei das Medium in der Kassette (14) mit dem Medizinprodukt (11) in Kontakt gerät und die Aufbereitung durchführt,
d) Abführen des Aufbereitungsmediums aus dem oder den Abführanschlüssen,
e) Verwerfen des abgeführten Aufbereitungsmediums, wobei die Temperatur des Aufbereitungsmediums während des gesamten Aufbereitungsprozesses 100°C nicht übersteigt.

Gegenstand der Erfindung ist ferner eine Anordnung zur Durchführung dieses Verfahrens.

## Beschreibung

Die Erfindung betrifft ein Verfahren zum maschinellen Aufbereiten eines wiederverwendbaren Medizinprodukts, welches keimarm oder steril zur Anwendung kommen soll, das Höhlräume, Kanäle und/oder Gleitflächen aufweist. Gegenstand der Erfindung ist ferner eine Anordnung zur Durchführung des Verfahrens.

Wiederverwendbare medizinische und chirurgische Instrumente müssen nach der Verwendung gereinigt, desinfiziert, sowie gegebenenfalls verpackt und sterilisiert werden. Bei üblichen chirurgischen Instrumenten kann dies beispielsweise durch Reinigen und thermische Desinfektion in einem Reinigungs- und Desinfektionsgerät (RDG) mit anschließender Dampfsterilisation bei Temperaturen oberhalb 120°C geschehen.

Diese Vorgehensweise ist zum einen aufwendig und zum anderen problematisch beispielsweise bei der Aufbereitung von Endoskopen, die thermolabile Materialien wie Gummi, Kunststoff oder dergleichen enthalten, und schlecht zu spülende Geometrien aufweisen.

Es ist daher beispielsweise in WO-A-2005/056060 bereits vorgeschlagen worden, Endoskope mit Vorrichtungen zu reinigen, bei denen Reinigungsmedium im Kreislauf durch die Endoskope geführt wird und dabei insbesondere auch die englumigen Höhlräume spült.

Der Erfindung liegt die Aufgabe zugrunde, ein Verfahren und eine Vorrichtung der eingangs genannten Art zu schaffen, das bzw. die eine besonders einfache, sorgfältige und schnelle Aufbereitung von Medizinprodukten ermöglicht.

Das erfindungsgemäße Verfahren weist folgende Schritte auf:
a) Einlegen des Medizinprodukts in eine verschließbare Kassette, die Anschlüsse für die Zu- und Abfuhr von Aufbereitungsmedium aufweist,
b) Verschließen der Kassette,
c) Zuführen von frischem Aufbereitungsmedium in den oder die Zuführanschlüsse der Kassette, wobei das Medium in der Kassette mit dem Medizinprodukt in Kontakt gerät und die Aufbereitung durchführt,
d) Abführen des Aufbereitungsmediums aus dem oder den Abführanschlüssen,
e) Verwerfen des abgeführten Aufbereitungsmediums, wobei die Temperatur des Aufbereitungsmediums während des gesamten Aufbereitungsprozesses 100°C nicht übersteigt.

Zunächst seien einige im Rahmen der Erfindung verwendete Begriffe erläutert.

Wiederverwendbare Medizinprodukte sind mehrfach verwendbare Instrumente und/oder Apparate, die in der Regel dem Medizinproduktegesetz unterfallen. Es kann sich insbesondere um minimalinvasive chirurgische (MIC) Instrumente, sowie starre oder flexible Endoskope handeln.

Die Medizinprodukte weisen häufig Hohlräume, Kanäle und/oder Gleitflächen auf. Diese Begriffe bezeichnen zusammenfassend Flächenanteile, die durch Besprühen oder Bespülen mit Aufbereitungsmedium von außen nicht ohne weiteres zugänglich sind. Beispielsweise kann es sich hier um die Schläuche oder Lumen (bspw. Arbeitskanäle) von Endoskopen oder einander zugewandte Gleitflächen beweglicher Teile, wie Scheren oder dergleichen handeln.

Bei dem erfindungsgemäßen Verfahren wird das Medizinprodukt in einem ersten Schritt in eine verschließbare Kassette eingelegt. Einlegen bedeutet, dass es dort in einer vorzugsweise definierten Position angeordnet wird. Zu diesem Zweck können in der Kassette entsprechende Halterungen vorgesehen sein. Die Kassette ist verschließbar. Es handelt sich somit um einen Behälter, in dem das Medizinprodukt nach dem Einlegen und Verschließen der Kassette vor Umwelteinflüssen geschützt ist. Die Kassette weist Anschlüsse für die Zu- und Abfuhr von Aufbereitungsmedien auf. Mittels dieser Anschlüsse kann die Kassette und das darin befindliche Medizinprodukt von den Aufbereitungsmedien durchströmt werden.

Bei der erfindungsgemäßen Aufbereitung wird für jeden Prozessschritt frisches Aufbereitungsmedium verwendet. Frisch bedeutet in diesem Zusammenhang, dass das Medium noch nicht mit einem Medizinprodukt zwecks Aufbereitung in Kontakt gewesen ist, insbesondere nicht (wie im Stand der Technik) im Kreislauf geführt wird. Das Medium gerät in der Kassette mit dem Medizinprodukt und dessen Hohlräumen, Kanälen und/oder Gleitflächen in Kontakt und führt die Aufbereitung durch. Zu diesem Zweck können Zuführanschlüsse der Kassette so ausgebildet sein, dass sie Aufbereitungsmedium gezielt in diese Hohlräume oder Kanäle lenken. Beispielsweise kann die Kassette innen einen oder mehrere Anschlüsse aufweisen, an den Hohlräume oder Schläuche des Endoskops angeschlossen werden und so bei der Aufbereitung gezielt von Aufbereitungsmedium durchströmt werden. Zusätzlich kann das Medizinprodukt beispielsweise von außen mit Aufbereitungsmedium bespült werden. Im Rahmen der Erfindung ist es bevorzugt, dass die Zu- und Abführanschlüsse der Kassette verschließbare Ventile aufweisen, so dass in der verschlossenen Kassette das darin befindliche Medizinprodukt keimdicht hygienisch gelagert werden kann. Bevorzugt ist es, wenn diese Ventile automatisch schließen, sobald die äußeren Zu- bzw. Abführanschlüsse von der Kassette gelöst werden.

Nach dem Aufbereiten wird das Aufbereitungsmedium aus dem oder den Abführanschlüssen abgeführt, d.h. aus der Kassette entfernt. Es wird unmittelbar danach verworfen, also keiner weiteren Verwertung im Rahmen des erfindungsgemäßen Verfahrens zugeführt.

Erfindungsgemäß handelt es sich somit um eine Reinigung, bei der das oder die Aufbereitungsmedien nach einmaliger Verwendung verworfen werden.

Erfindungsgemäß ist vorgesehen, dass die Temperatur des Aufbereitungsmediums während des gesamten Aufbereitungsprozesses 100°C nicht übersteigt. Das erfindungsgemäße Verfahren ist somit besonders vorteilhaft verwendbar für thermolabile Medizinprodukte, die nicht mit der im Stand der Technik üblichen Dampfsterilisation (Temperaturen oberhalb 120°C) behandelt werden können.

Die durch das erfindungsgemäße Verfahren geringe thermische Belastung ist vorteilhaft auch bei solchen Medizinprodukten anwendbar, die eine höhere Temperatur zwar prinzipiell vertragen, deren Lebensdauer aber durch höhere Temperaturen wie beispielsweise bei der Dampfsterilisation eingeschränkt wird. Durch das erfindungsgemäße Verfahren können auch solche Produkte schonend aufbereitet und dementsprechend häufiger verwendet werden.

Im Stand der Technik ist bei der maschinellen Aufbereitung von Endoskopen immer ein mehrfaches Durchspülen der Endoskope mit im Kreislauf umgewälztem Aufbereitungsmedium vorgesehen. Dahinter steht der Gedanke, dass das Aufbereiten mit möglichst geringen Mengen des Aufbereitungsmediums geschehen soll.

Die Erfindung hat erkannt, dass diese Kreislaufreinigung nachteilig ist. Sie führt insbesondere dazu, dass mögliche lokale Kontaminationen im Endoskop durch den Kreislauf auf das gesamte System und damit Endoskop übertragen werden. Es erfolgt also eine fortlaufende Rekontamination. Die erfindungsgemäße Reinigung bewirkt demgegenüber, dass das Aufbereitungsmedium Kontaminationen aufnimmt und diese durch das Verwerfen sofort abgeführt werden. Eine Rekontamination kann nicht stattfinden. Überaschenderweise kann erfindungsgemäß trotz gegenüber der Kreislaufreinigung deutlich geringerer Volumina von durch das Endoskop geführten Aufbereitungsmedien eine wirkungsvolle und schnelle Aufbereitung erfolgen.

Die erfindungsgemäße Aufbereitung kann eine Reinigung und Desinfektion umfassen. Die Begriffe Reinigung und Desinfektion sind definiert im Entwurf DIN EN ISO 15883-4 in der Fassung vom Juni 2005.

Die Sterilisation ist definiert in der Norm EN 556, derzufolge die theoretische Wahrscheinlichkeit, dass sich ein lebensfähiger Mikroorganismus auf dem Produkt befindet, kleiner oder gleich 1 bei 10⁶ Produkten sein muss.

Somit verlangt eine Desinfektion eine relative Verminderung (relativ zur Ausgangskontamination) um bestimmte Größenordnungen. Der Begriff der Sterilisation verlangt unabhängig vom Ausgangsniveau der Kontamination eine Verminderung aller lebensfähigen Keime auf ein absolut definiertes Höchstniveau.

Bevorzugte Temperaturen der Aufbereitungsmedien liegen bei maximal 90°C, weiter vorzugsweise maximal 80°C, weiter vorzugsweise maximal 70°C, weiter vorzugsweise maximal 60°C. Bevorzugte Untergrenzen sind 20 bzw. 30 °C. Ein besonders bevorzugter Temperaturbereich ist 30 bis 55°C.

Erfindungsgemäß ist es bevorzugt, das die Aufbereitungsschritte c) bis e) des Anspruchs 1 wenigstens zweimal nacheinander unter Verwendung von wenigstens zwei unterschiedlichen Aufbereitungsmedien durchgeführt werden. Die Aufbereitungsmedien sind bevorzugt flüssig.

Ein erstes Aufbereitungsmedium kann eine tensidhaltige Reinigungslösung sein.

Die tensidhaltige Reinigungslösung kann Enzyme als reinigungsaktive Bestandteile enthalten. Sie ist bevorzugt neutral oder schwach alkalisch, kann beispielsweise einen pH-Wert von 6 bis 12, vorzugsweise 6 bis 11, weiter vorzugsweise 7 bis 11, weiter vorzugsweise 8 bis 10,5, weiter vorzugsweise 9 bis 10,5, aufweisen.

Ein zweites Aufbereitungsmedium kann Desinfektionsmittel enthalten. Als Desinfektionsmittel können Oxidationsmittel wie z.B. peroxidische Wirkstoffe (Peressigsäure, Percarbonate, Ozon oder dergleichen). Beispielsweise kann Wasserstoffperoxid verwendet werden. Bevorzugt ist eine 10-30%ige Wasserstoffperoxidlösung.

Im Rahmen der Erfindung ist es besonders bevorzugt, dass die Aufbereitungsmedien anwendungsfertig zubereitet aus Vorratsbehältern entnommen werden, in denen sie in der vorgesehenen Anwendungskonzentration bereitgestellt werden. Bei dieser besonders bevorzugten Ausführungsform erfolgt also während des Prozesses keine Verdünnung von Konzentraten (beispielsweise mit Wasser), sondern die Aufbereitungsmedien werden von vornherein in der Anwendungskonzentration bereitgestellt und verwendet. Dies hat verschiedene Vorteile.

Für eine ordnungsgemäße Aufbereitung müssen die Aufbereitungsmedien keimfrei/steril sein. Dies ist gewährleistet, wenn man die Medien entsprechend keimarmen oder sterilen Vorratsbehältern entnimmt. Verdünnt man jedoch Konzentrate mit Wasser, kann das verwendete Wasser eine Quelle zusätzlicher Kontaminationen sein, die nicht oder nur schwer kontrollierbar ist. Durch den Verzicht auf einen Wasseranschluss kann das erfindungsgemäße Verfahren zu dem in wesentlich einfacheren und kompakteren Anordnungen durchgeführt werden, wie später noch zu erläutern sein wird.

Bei dieser bevorzugten Ausführungsform der Erfindung findet keine Verwendung von Leitungswasser für eine zwischenspülung zwischen zwei Aufbereitungsschritten oder eine Vor- bzw. Nachspülung statt. Es ist dann bevorzugt, wenn ein nachgeschaltetes Aufbereitungsmedium (beispielsweise die Desinfektionslösung) gleichzeitig als Spülmedium zur Entfernung etwaiger Reste (Tensidreste oder dergleichen) aus dem ersten Aufbereitungsschritt ist. In diesem Zusammenhang ist die Verwendung von wasserstoffperoxidhaltigen Desinfektionsmitteln bevorzugt, da dann gleichzeitig ein Ausspülen von Reinigungsmedien erfolgt, und Wasserstoffperoxid selbst keinerlei Rückstände hinterlässt. Im Rahmen der Erfindung ist es gleichfalls möglich, zwischen zwei Aufbereitungsschritten oder nach dem letzten Aufbereitungsschritt (vorzugsweise nach der Desinfektion) eine Zwischen- bzw. Nachspülung mit Spülmedium vorzunehmen. Grundsätzlich ist es im Rahmen der Verfahrenserfindung möglich, zu diesem Zweck Leitungswasser zu verwenden, das gegebenenfalls (vorzugsweise keimfrei) aufbereitet wird. Im Rahmen der Erfindung ist es jedoch bevorzugt, dass ein solches Spülmedium wie beispielsweise voll entsalztes und keimfreies Wasser ebenfalls anwendungsfertig aus einem Vorratsbehälter entnommen wird. Im Rahmen der Erfindung werden - sofern Spülmedium überhaupt benötigt wird - nur sehr geringe Mengen Spülmedium verwendet. Vorzugsweise wird für einen etwaigen Zwischen- oder Nachspülschritt nicht mehr Spülmedium verwendet, als für den vorherigen Aufbereitungsschritt.

Im Rahmen der Erfindung ist es besonders bevorzugt, dass sämtliche verwendeten Aufbereitungsmedien anwendungsfertig präpariert aus Vorratsbehältern entnommen werden, in denen sie in der vorgesehenen Anwendungskonzentration bereitgestellt werden.

Die Zufuhr von Medien aus Vorratsbehältern (ggf. gemischt mit Leitungswasser) erfolgt mittels Dosiereinrichtungen, die geeignete Pumpen, Ventile und dergleichen aufweisen. Die Dosiereinrichtungen können zu einer Dosiereinheit zusammengefasst sein. Eine solche Dosiereinheit kann Teil der Basisstation mit den Vorratsbehältern sein. Alternativ ist es möglich, die Dosiereinheit als Teil der verschließbaren Kassette auszubilden. Bei einer bevorzugten Ausführungsform ist die Dosiereinheit eine separate Einheit, die auf die Kassette aufsetzbar ist. Bei dieser Ausführungsform der Erfindung enthält die Dosiereinheit bevorzugt Druck- und Temperatursensoren für das Medium oder die Medien sowie Dosierventile. Die Pumpen für die Medien sind bevorzugt in der Basiseinheit angeordnet. Mittels der Dosierventile der Dosiereinheit wird auch die Verteilung der Medien auf die unterschiedlichen Anschlüsse der Kassette (Spülung der verschiedenen Kanäle der Instrumente sowie Besprühen oder Bespülen der Außenoberfläche) gesteuert.

Erfindungsgemäß kann im Schritt c) eine Variation des Strömungsprofils des Aufbereitungsmediums erfolgen. Eine solche Variation des Strömungsprofils kann beispielsweise Druckwechsel beinhalten, beispielsweise durch Verwendung einer Pumpe, die das Medium diskontinuierlich bzw. stoßartig fördert.

In einer weiteren Ausführungsform kann die Variation des Strömungsprofils durch die Verwendung eines wenigstens zweiphasigen Aufbereitungsmediums erfolgen. Insbesondere kann ein flüssiges Aufbereitungsmedium gasförmige Einschlüsse (beispielsweise in Form steriler Luft) enthalten. Solche Gaseinschlüsse fördern das mechanische Abtragen von Verunreinigungen von der Oberfläche des Medizinproduktes. Bei der Verwendung solcher Gaseinschlüsse kann man auch Kavitationseffekte an der Oberfläche des Medizinproduktes hervorrufen, die eine besonders starke mechanische Entfernung von Verunreinigungen bewirken.

Die Erfindung hat erkannt, dass bei der verlorenen Reinigung mit fertig zubereiteten Aufbereitungsmedien sehr geringe Volumina zur vollständigen Reinigung/Desinfektion/Sterilisierung der Medizinprodukte, insbesondere Endoskope, ausreichen. Bevorzugt ist das Gesamtvolumen jedes verwendeten Aufbereitungsmediums in den Schritten c) bis e) 2000 ml oder weniger, weiter vorzugsweise 1000 ml oder weniger.

Gegenstand der Erfindung ist ferner eine Anordnung zur Durchführung des Verfahrens, die aufweist:
a) eine verschließbare Kassette, die zur Aufnahme des Medizinproduktes ausgebildet ist und die Anschlüsse für die Zu- und Abfuhr von Aufbereitungsmedium aufweist,
b) eine Basisstation, an die wenigstens eine Kassette angeschlossen werden kann und die Zu- und Abfuhrleitungen für Aufbereitungsmedium aufweist, die mit den Anschlüssen für die Zu- und Abfuhr von Aufbereitungsmedium der Kassette verbindbar sind,
c) die Zufuhrleitungen für Aufbereitungsmedium sind unmittelbar und ausschließlich mit Vorratsbehältern für Aufbereitungsmedien verbunden,
d) die Abfuhrleitungen für Aufbereitungsmedium sind unmittelbar und ausschließlich mit einer Einrichtung zum Verwerfen des benutzten Aufbereitungsmediums verbunden.

Kennzeichnend für die Vorrichtung ist, dass sie ausschließlich zur verlorenen Reinigung ausgebildet ist und weder Einrichtungen zum Führen von Reinigungsmedium im Kreislauf aufweist noch eine Verbindung mit einer Wasserzufuhr zum Verdünnen oder Spülen von Aufbereitungskonzentraten vorgesehen ist. Die Formulierung, dass die Zufuhrleitungen für Aufbereitungsmedium unmittelbar und ausschließlich mit Vorratsbehältern für Aufbereitungsmedien verbunden sind, bedeutet, dass keinerlei Einrichtung vorgesehen ist, die ein Verdünnen der Aufbereitungsmedien ermöglichen würde. Im Rahmen der Erfindung bedeutet "unmittelbar und ausschließlich", dass keine Abzweigungen für eine Verdünnung mit flüssigem Medium (Wasser) vorgesehen sind. Die Formulierung schließt selbstverständlich nicht aus, dass Steuerungselemente für den Durchfluss verbunden sind wie beispielsweise Pumpen, Ventile, Meß- und Überwachungseinrichtungen oder auch Einrichtungen zur Konditionierung der Medien wie beispielsweise eine Heizung. Die Formulierung schließt ferner nicht aus, dass insbesondere Einrichtungen zur Zufuhr eines weiteren gasförmigen Aufbereitungsmediums in das flüssige Aufbereitungsmedium vorhanden sind. Wesentlich ist im Rahmen dieser Ausführungsform der Erfindung, dass kein Wasseranschluss erforderlich bzw. vorgesehen ist, der gleichzeitig eine Aufbereitung von zu Verdünnungszwecken zugeführtem Leitungswasser erforderlich machen würde. Die geschilderte Ausführungsform der Erfindung benötigt somit weder einen externen Wasseranschluss noch bedarf es aufwendiger Einrichtungen zur Aufbereitung von Leitungswasser. Die Abfuhrleitungen sind unmittelbar und ausschließlich mit einer Einrichtung zum Verwerfen des benutzten Aufbereitungsmediums verbunden. Dies bedeutet, dass das benutzte Aufbereitungsmedium nicht im Kreislauf einer Wiederverwendung zugeführt werden kann. Es wird beispielsweise unmittelbar dem Abfluss oder einem Auffangbehälter für benutztes Aufbereitungsmedium zugeführt.

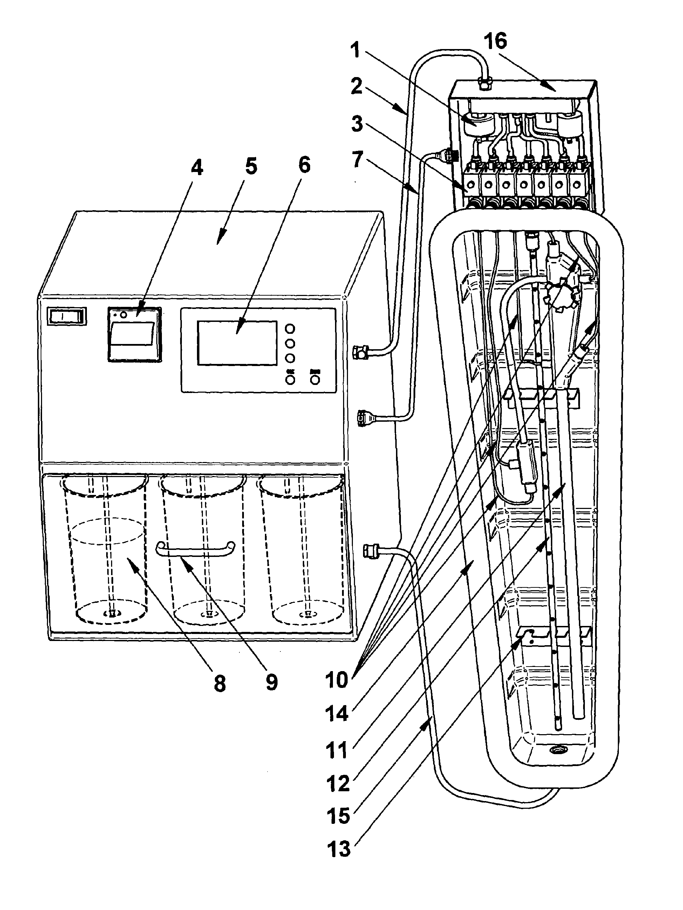

Ausführungsbeispiele der Erfindung werden im folgenden erläutert. Die einzige Zeichnung zeigt eine Anordnung zur Durchführung des erfindungsgemäßen Verfahrens.

In einer verschließbaren Kunststoffkassette 14 sind Halterungen 13 für ein Endoskop 11 angeordnet. Die durchströmbaren Lumina des Endoskops sind über Anschlussschläuche 10 verbunden mit den Anschlüssen für die Zufuhr von Aufbereitungsmedium aus einer Dosiereinheit 16. Der Ablauf von Aufbereitungsmedium findet über einen Anschluss im Boden der Kassette 14 statt, an dem ein Ablaufschlauch 15 angeschlossen ist.

Auf die Zuführanschlüsse für Aufbereitungsmedium der Kassette 14 kann über Leitungsverbindungen eine Dosiereinheit 16 aufgesetzt werden, die Druck- und Temperatursensoren 1 sowie Dosierventile 3 enthält. Sie ist über einen Zulaufschlauch 2 sowie elektrische Mess- und Steuerleitungen 7 mit einer Basiseinheit 5 verbunden. In der Basiseinheit 5 befinden sich Vorratsbehälter 8 mit einem oder verschiedenen Aufbereitungsmedien. Die Vorratsbehälter 8 stehen in einem mit einer Tür 9 verschließbaren Aufbewahrungsraum.

Die Basiseinheit 5 weist ferner eine Prozesssteuerung (nicht dargestellt) ein Bedien-/Anzeigepanel 6 sowie einen Protokolldrucker 4 auf.

### Beispiel 1:

### Herstellung eines Reinigungs- und Desinfektionsmittels

Es wird eine Reinigungsmittellösung der folgenden Zusammensetzung hergestellt

| **Inhalt** | **Gewichts-%** |
|---|---|
| Triethanolamin | 2,5 |
| Propylenglykol | 5,0 |
| Protease | 0,17 |
| Amylase | 0,17 |
| Ethoxylierter Fettalkohol | 0,17 |
| Rest Wasser | Auf 100 Gewichts-% |

Als Desinfektionslösung wurde eine 30%ige Wasserstoffperoxidlösung in vollentsalztem Wasser hergestellt.

### Beispiel 2:

Dieses Beispiel beschreibt die Durchführung des erfindungsgemäßen Reinigungsverfahrens.

In die Kassette 14 werden die zu reinigenden Endoskope eingelegt und die durchspülbaren Kanäle werden mittels der Schläuche 10 mit den Zufuhranschlüssen in der Wandung der Kassette verbunden. Die Zuführanschlüsse der Kassette 14 sind mit der Dosiereinheit 16 verbunden. Der Abführanschluss ist mit dem Ablaufschlauch 15 verbunden. In der Basiseinheit 5 werden die vorstehend beschriebenen Reinigungs- und Desinfektionslösungen bereitgestellt.

Zunächst wird Reinigungslösung in einem in der Basiseinheit 5 angeordneten nicht dargestellten Durchlauferhitzer auf etwa 40°C erwärmt und mittels der Dosiereinrichtung 3 durch die Anschlussschläuche 10 in die Kanäle des Endoskops gepumpt. Zusätzlich erfolgt mittels des Sprührohrs 12 ein Ansprühen der Außenflächen des Endoskops.

Nach dem Befüllen/Durchspülen aller Kanäle wird die Reinigungslösung 1 min in Ruhe einwirken gelassen, anschließend wird für einen Zeitraum von 3 s Reinigungslösung nachdosiert. Die Förderleistung der verwendeten Pumpe liegt bei 650 ml/min, bei einer Pumpzeit von 3 s werden somit ungefähr 32 ml Reinigungslösung nachdosiert. Diese Menge reicht aus, um die in den erstbefüllten Endoskopkanälen enthaltene Flüssigkeitsmenge (bei üblichen Endoskopen ungefähr 20 ml) vollständig auszutauschen.

Der Zyklus 1 min in Ruhe Einwirken, 3 s Pumpen zur Erneuerung der Reinigungslösung wird noch zweimal wiederholt. Zum Abschluss des ersten Reinigungszyklus wird die Reinigungslösung noch einmal 1 min einwirkengelassen.

Anschließend wird als zweiter Reinigungszyklus der gesamte vorstehend beschriebene Ablauf mit insgesamt vier durch Pumpintervallen unterbrochenen 1 min Einwirkzeiten wiederholt mit einer Reinigungslösung, die im Durchlauferhitzer auf 50°C erwärmt wird.

Nach diesem zweiten Reinigungszyklus werden die Kanäle entleert. Die Reinigungslösung kann entweder mit Desinfektionslösung ausgespült oder in einem Zwischenschritt mit Luft ausgespült werden.

Im nächsten Schritt wird die Desinfektionslösung im Durchlauferhitzer auf 45°C erwärmt und in die Kanäle des Endoskops eingebracht sowie von außen mittels des Sprührohrs 12 auf die Oberflächen des Endoskops aufgesprüht. Die Dauer des erstmaligen Einsprühens bzw. Befüllens der Kanäle beträgt etwa 10 s, in dieser Zeit werden insgesamt etwa 10 ml Desinfektionslösung in die Kanäle des Endoskops eingebracht bzw. auf die Oberflächen des Endoskops aufgesprüht.

Im Anschluss daran wird die Desinfektionslösung 50 s in Ruhe einwirken gelassen. Der genannte Zyklus (10 s Pumpen/Einsprühen, 50 s in Ruhe Einwirkenlassen) wird noch 9x wiederholt, so dass sich insgesamt eine Desinfektionszeit von etwa 10 min bei einem Verbrauch von 1 1 Desinfektionslösung ergibt.

Nach dem Einwirken der Desinfektionslösung wird diese mit auf 55°C erwärmter steril-gefilterter Luft ausgeblasen und das Endoskop innen und außen mit dieser erwärmten Luft während eines Zeitraums von 10 min getrocknet.

Aus der Kassette 14 abfließende gebrauchte Reinigungs- oder Desinfektionslösung wird mittels des Ablaufschlauchs 15 in die Basisstation 5 zurückgeführt und dort entweder in einem regelmäßig zu leerenden Abfallbehälter aufbewahrt oder unmittelbar einem Abwasseranschluss zugeführt.

Das Endoskop kann anschließend aus der Kassette 14 zur Verwendung entnommen werden, alternativ kann es darin bis zur vorgesehenen Verwendung gelagert werden. Durch Abnehmen des Zulaufschlauchs 2 von der Dosiereinheit 16 und des Ablaufschlauchs 15 von der Kassette schließen automatisch Ventile in den Zu-/Ablauföffnungen der Kassette 14, so dass der Zustand im Inneren der Kassette gewahrt bleibt.

Der Verbrauch an Reinigungs- und Desinfektionslösung für den gesamten Programmablauf beträgt bei diesem Ausführungsbeispiel jeweils 1 1.

Abhängig von der Größe der Kassette 14 und der Zahl und Art der zu reinigenden Endoskope kann der Verbrauch an Reinigungslösung variieren.

## Patentansprüche

1. Verfahren zum maschinellen Aufbereiten eines wieder verwendbaren Medizinprodukts (11), mit den Schritten:
a) Einlegen des Medizinprodukts (11) in eine verschließbare Kassette (14), die Anschlüsse für die Zu- und Abfuhr von Aufbereitungsmedium aufweist,
b) Verschließen der Kassette (14),
c) Zuführen von frischem Aufbereitungsmedium in den oder die Zuführanschlüsse der Kassette (14), wobei das Medium in der Kassette (14) mit dem Medizinprodukt (11) in Kontakt gerät und die Aufbereitung durchführt,
d) Abführen des Aufbereitungsmediums aus dem oder den Abführanschlüssen,
e) Verwerfen des abgeführten Aufbereitungsmediums, wobei die Temperatur des Aufbereitungsmediums während des gesamten Aufbereitungsprozesses 100°C nicht übersteigt.

2. Verfahren nach Anspruch 1, **dadurch gekennzeichnet, dass** das Medizinprodukt (11) Gleitflächen und/oder durchströmbare Hohlräume und/oder Kanäle aufweist.

3. Verfahren nach Anspruch 1 oder 2, **dadurch gekennzeichnet, dass** die Temperatur des Aufbereitungsmediums während des gesamten Aufbereitungsprozesses 90°C, vorzugsweise 80°C, weiter vorzugsweise 70°C, weiter vorzugsweise 60°C nicht übersteigt.

4. Verfahren nach einem der Ansprüche 1 bis 3, **dadurch gekennzeichnet, dass** die Medizinprodukte nicht dampfsterilisierbar sind.

5. Verfahren nach einem der Ansprüche 1 bis 4, **dadurch gekennzeichnet, dass** die Aufbereitung eine Reinigung und eine Desinfektion umfasst.

6. Verfahren nach Anspruch 5, **dadurch gekennzeichnet, dass** die Aufbereitung zusätzlich eine Sterilisation umfasst.

7. Verfahren nach einem der Ansprüche 1 bis 4, **dadurch gekennzeichnet, dass** die Schritte c) bis e) wenigstens zweimal nacheinander unter Verwendung von wenigstens zwei unterschiedlichen Aufbereitungsmedien durchgeführt werden.

8. Verfahren nach Anspruch 7, **dadurch gekennzeichnet, dass** ein erstes Aufbereitungsmedium eine tensidhaltige Reinigungslösung ist.

9. Verfahren nach Anspruch 8, **dadurch gekennzeichnet, dass** die tensidhaltige Reinigungslösung Enzyme enthält.

10. Verfahren nach Anspruch 8, **dadurch gekennzeichnet, dass** die tensidhaltige Reinigungslösung einen pH-Wert von 6 bis 12, vorzugsweise 6 bis 11, weiter vorzugsweise 7 bis 11, weiter vorzugsweise 8 bis 10,5, weiter vorzugsweise 9 bis 10,5, aufweist.

11. Verfahren nach einem der Ansprüche 7 bis 10, **dadurch gekennzeichnet, dass** ein zweites Aufbereitungsmedium Desinfektionsmittel enthält.

12. Verfahren nach Anspruch 11, **dadurch gekennzeichnet, dass** die Desinfektionsmittel Peroxide, vorzugsweise Wasserstoffperoxid, umfassen.

13. Verfahren nach einem der Ansprüche 1 bis 12, **dadurch gekennzeichnet, dass** die Aufbereitungsmedien anwendungsfertig präpariert aus Vorratsbehältern entnommen werden, in denen sie in der vorgesehenen Anwendungskonzentration bereitgestellt werden.

14. Verfahren nach Anspruch 13, **dadurch gekennzeichnet, dass** sämtliche Aufbereitungsmedien anwendungsfertig präpariert aus Vorratsbehältern entnommen werden, in denen sie in der vorgesehenen Anwendungskonzentration bereitgestellt werden.

15. Verfahren nach einem der Ansprüche 1 bis 14, **dadurch gekennzeichnet, dass** in Schritt c) eine Variation des Strömungsprofils des Aufbereitungsmediums erfolgt.

16. Verfahren nach Anspruch 15, **dadurch gekennzeichnet, dass** die Variation des Strömungsprofils Druckwechsel beinhaltet.

17. Verfahren nach Anspruch 15 oder 16, **dadurch gekennzeichnet, dass** die Variation des Strömungsprofils durch ein wenigstens zweiphasiges Aufbereitungsmedium erfolgt.

18. Verfahren nach Anspruch 17, **dadurch gekennzeichnet, dass** das zweiphasige Aufbereitungsmedium gasförmige Einschlüsse in flüssiger Phase aufweist.

19. Verfahren nach Anspruch 17 oder 18, **dadurch gekennzeichnet, dass** die Variation des Strömungsprofils Kavitationseffekte umfasst.

20. Verfahren nach einem der Ansprüche 1 bis 19, **dadurch gekennzeichnet, dass** das Gesamtvolumen jedes verwendeten Aufbereitungsmediums in den Schritten c) bis e) 1.000 ml oder weniger beträgt.

21. Verfahren nach einem der Ansprüche 1 bis 20, **dadurch gekennzeichnet, dass** die Temperatur der Aufbereitungsmedien in Schritt c) zwischen 20 und 80°C, vorzugsweise zwischen 30 und 55°C liegt.

22. Anordnung zur Durchführung des Verfahrens nach einem der Ansprüche 1 bis 21, **dadurch gekennzeichnet, dass** sie aufweist:
a) eine verschließbare Kassette (14), die zur Aufnahme des Medizinproduktes (11) ausgebildet ist und die Anschlüsse für die Zu- und Abfuhr von Aufbereitungsmedium aufweist,
b) eine Basisstation (5), an die wenigstens eine Kassette (14) angeschlossen werden kann und die Zu- und Abfuhrleitungen (2, 15) für Aufbereitungsmedium aufweist, die mit den Anschlüssen für die Zu- und Abfuhr von Aufbereitungsmedium der Kassette (14) verbindbar sind,
c) die Zufuhrleitungen (2) für Aufbereitungsmedium sind unmittelbar und ausschließlich mit Vorratsbehältern (8) für Aufbereitungsmedien verbunden,
d) die Abfuhrleitungen (15) für Aufbereitungsmedium sind unmittelbar und ausschließlich mit einer Einrichtung zum Verwerfen des benutzten Aufbereitungsmediums verbunden.
